# FASCICULE DE BREVET EUROPEEN

(11) **EP 1 802 310 B1**
(45) Date de publication et mention de la délivrance du brevet: **17.12.2014**
(21) Numéro de dépôt: 05804720.0
(22) Date de dépôt: 14.10.2005
(51) Int. Cl.: A61K 31/52, A61P 11/00

(54) **UTILISATION DE DERIVES DE PURINES POUR LA FABRICATION DE MEDICAMENTS POUR LE TRAITEMENT DE LA MUCOVISCIDOSE ET DE MALADIES LIEES A UN DEFAUT D'ADRESSAGE DES PROTEINES DANS LES CELLULES**
VERWENDUNG VON PURIN-DERIVATEN ZUR HERSTELLUNG VON MEDIKAMENTEN ZUR BEHANDLUNG VON MUKOVISZIDOSE UND KRANKHEITEN IM ZUSAMMENHANG MIT PROTEINADRESSIERFEHLERN IN ZELLEN
USE OF PURINE DERIVATIVES FOR THE PRODUCTION OF MEDICAMENTS FOR THE TREATMENT OF MUCOVISCIDOSIS AND DISEASES RELATED TO PROTEIN ADDRESSING ERRORS IN CELLS

(30) Priorité: 15.10.2004 FR 0410958
(43) Date de publication de la demande: 04.07.2007
(73) Titulaire: Manros Therapeutics, 29680 Roscoff (FR)
(72) Inventeur: BECQ, Frédéric, F-86280 Poitiers (FR); MEIJER, Laurent, F-29680 Roscoff (FR)
(74) Mandataire: Nony
(86) Numéro de dépôt international: PCT/FR2005/002557
(87) Numéro de publication internationale: WO 2006/042949

(56) Documents cités:
- FR-A- 2 741 881
- VESELY J ET AL: "INHIBITION OF CYCLIN-DEPENDENT KINASES BY PURINE ANALOGUES" EUROPEAN JOURNAL OF BIOCHEMISTRY, BERLIN, DE, vol. 224, no. 2, 1 septembre 1994 (1994-09-01), pages 771-786, XP000576956 ISSN: 0014-2956 cité dans la demande
- BECQ F ET AL: "Pharmacological interventions for the correction of ion transport defect in cystic fibrosis" EXPERT OPINION ON THERAPEUTIC PATENTS 2004 UNITED KINGDOM, vol. 14, no. 10, 2004, pages 1465-1483, XP002330339 ISSN: 1354-3776
- JACOBSON K A ET AL: "STIMULATION BY ALKYLXANTHINES OF CHLORIDE EFFLUX IN CFPAC-1 CELLS DOES NOT INVOLVE A1 ADENOSINE RECEPTORS" BIOCHEMISTRY, AMERICAN CHEMICAL SOCIETY. EASTON, PA, US, vol. 34, no. 28, 18 juillet 1995 (1995-07-18), pages 9088-9094, XP000565691 ISSN: 0006-2960

## Description

L'invention vise l'utilisation de dérivés de purines pour fabriquer des médicaments capables de restaurer l'adressage de protéines du réticulum endoplasmique vers les membranes plasmiques. Elle vise tout particulièrement le traitement de la mucoviscidose.

L'article s'intitulant pharmacological interventions for the correction of ion transport defect in cystic fibrosis », expert opinion on therapeutic patents 2004 United Kingdom, vol. 14, n°10, 2004, pages 1465-1483 de Becq et al.et l'article s'intitulant "stimulation by alkylxanthines of chloride efflux in CFPAC-1 cells does not involve A1 adenosine receptors", biochemistry, American Chemical Society, Easton, PA, US, vol.34, n°28, 18 juillet 1995, pages 9088-9094 de Jacobson K A et al, divulguent des dérivés de la xanthine mais pour une application dans le traitement de la mucoviscidose mais ne divulguent pas de dérivés de purines selon la présente Invention.

La mucoviscidose (CF : *Cystic Fibrosis*) est la maladie génétique récessive, autosomique, létale, la plus répandue dans les populations européennes et nord américaines. Le gène CF (locus 7q31) code pour la protéine trans-membranaire nommée CFTR (pour *Cystic Fibrosis Transmembrane* Conductance *Regulator*). Des mutations du gène CF provoquent un transport anormal d'eau et d'électrolytes à travers les membranes cellulaires de divers organes comme les poumons, les glandes sudoripares, l'intestin et le pancréas exocrine. Bien qu'il existe plus de 1000 mutations de la protéine CFTR, la mutation la plus fréquente (70 % des patients) est la délétion d'une phénylalanine. dans le domaine NBF1 en position 508 (delF508). La principale cause de mortalité des patients CF est liée à cette délétion et conduit à des infections ou à une insuffisance pulmonaire provoquées par une augmentation de la viscosité du mucus. Cette viscosité entraîne l'occlusion des voies respiratoires et favorise les infections par les bactéries opportunistes. Une aggravation est de plus constatée au niveau digestif et notamment pancréatique (patient pancréatique-insuffisant). La protéine CFTR est une glycoprotéine de 1480 acides aminés, appartenant à la superfamille des transporteurs membranaires ABC. CFTR est un canal chlorure localisé dans la membrane plasmique apicale des cellules épithéliales pulmonaires chez les individus sains.

CFTR est responsable du transport trans-épithélial d'-veau et d'électrolytes et permet chez un individu sain l'hydratation des voies aériennes pulmonaires.

Chez les patients CF, homozygotes pour la mutation delF508, et plus généralement pour les mutations de classe II (mutations produisant une protéine absente de la membrane cellulaire), cette protéine est absente des membranes plasmiques du fait d'un mauvais adressage de la protéine qui est retenue dans le réticulum endoplasmique (RE). L'hydratation des voies aériennes pulmonaires n'est plus fonctionnelle dans de cas. La délétion delF508 perturbe le repliement du domaine NBF1 et empêche la maturation complète de la protéine qui est donc dégradée très tôt au cours de sa biosynthèse. Cependant, si la protéine delF508 atteint la membrane, elle fonctionne comme un canal chlorure.

Une des clés d'un traitement de cette maladie consiste donc en un ré-adressage de delF508 vers la membrane plasmique des cellules au niveau de laquelle l'activité de transport de delF508 peut être stimulée par des agonistes physiologiques.

De manière surprenante, les inventeurs ont mis en évidence que des dérivés connus notamment pour leur effet anti-prolifératif étaient capables d'activer le CFTR sauvage et des formes mutées et de provoquer une re-localisation membranaire de la protéine de1F508-CFTR restaurant ainsi sa capacité de transport trans-membranaire. De manière générale, ces dérivés sont capables de restaurer un défaut d'adressage des protéines dans les cellules.

De plus, ces dérivés présentent l'intérêt d'une innocuité élevée.

Une nouvelle utilisation de ces dérivés est décrite pour fabriquer des médicaments pour le traitement de la mucoviscidose et de maladies liées à un défaut d'adressage des protéines dans les cellules.

Les dérivés. utilisés sont des purines substituées en positions 2, 6 et 9.

Des dérivés de ce type ont été décrits en tant qu'inhibiteurs de kinases.

De tels dérivés sont notamment décrits dans le brevet FR 2 741 881 et les brevets et demandes de brevets correspondant au WO 97/20842, ou dans l'article de Vesely et al, 1994, Eur.J.Biochem., 224,771-786 II, la 2-(2,hydroxyéthylamino)-6-benzylamino-9-méthylpurine, appelée communément olomoucine,

l'entrant pas dans le champ de l'invention.

Sont notamment décrits des dérivés répondant à la formule I 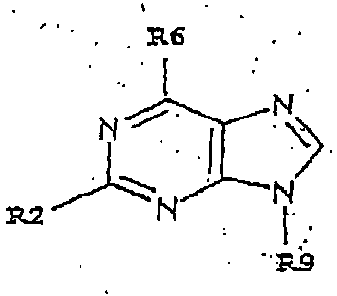 dans laquelle,
- R2, R6 et R9, identiques ou différents les uns des autres, représentent un atome d'halogène, un radical R-NH-R-NH-NH-, NH₂-R'-NH- ou R-NH-R'-NH-, dans lequel R représente un radical alkyle à chaîne droite ou ramifiée, saturé ou insaturé, un radical aryle ou cycloalkyle, ou un hétérocycle, et R' un groupe alcoylène à chaîne droite ou ramifiée, saturé ou insaturé, ou un groupe arylène ou cycloalcoylène, R et R' renfermant chacun de 1 à 8 atomes de carbone, et étant substitués, le cas échéant, par un ou plusieurs groupes -OH, halogène, amino ou alkyle,
- R2 prouvant, en outre, représenter un hétérocycle portant, le cas échéant, un radical alkyle à chaîne droite ou lamifiée saturé ou insaturé, un radical aryle ou cycloaryle, ou un hétérocycle, éventuellement substitué par un ou plusieurs groupes -OH, halogène, amino ou alkyle,
- R9 pouvant, en outre, représenter un radical alkyle à chaîne droite ou ramifiée, saturé ou insaturé, un radical aryle ou cycloalkyle,
- R2 et R9 pouvant représenter en outre un atome d'hydrogène, à l'exception de l'olomoucine.

En particulier est décrite l'utilisation de dérivés de formule I ci-dessus,dans laquelle R2 est choisi parmi les radicaux 3-hydroxypropylamino, 1-éthyl-2-hydroxyéthylamino, 5-hydroxypentylamino, 1-D,L hydroxyméthylpropylamino, aminoéthylamino, 2-bis (hydroxyéthyl)amino, 2- hydroxypropylamino, 2-hydroxyéthylamino, chloro, R-hydroxyméthyl-pyrrolidinyl, benzylaminohydroxyéthyl, R,S-aminohydroxyhexyl, S amino-2-phénylhydroxyéthyl, R amino-3-phénylhydroxypropyl, R,S aminohydroxypentyl, R aminohydroxypropyl, S aminohydroxypropyl, R(-) N-pyrrolidine hydroxyméthyl,
R9 est choisi parmi méthyl, isopropyl ou cyclopentyl, et
R6 est choisi parmi.
- benzylamino, 3-iodo-benzylamino, ou isopenténylamino, lorsque R9 représente un radical isopropyle,
- cyclohexylamino, cyclométhylamino, ou 3- hydroxybenzylamino, lorsque R9 représenté un radical méthyl, et
- benzylamino, lorsque R9 représente un radical cyclopentyl.

Ces dérivés correspondent aux isomères optiques et aux mélanges racémiques, le cas échéant aux isomères géométriques.

L'invention vise en particulier l'utilisation de la 2- (R, S) (1-éthyl-2-hydroxyéthylamino)-6-benzylamino-9-isopropylpurine ou de la 2-(R)-(1-éthyl-2-hydroxyéthylamino)-6-benzylamino-9-isopropylpurine, ou encore de la 2-(S) (1-éthyl-2-hydroxyéthylamino)-6-benzylamino-9-isopropylpurine, dans le traitement de la mucoviscidose.

Lesdits dérivés de purines, sont capables de restaurer l'adressage de la protéine CFTR vers les membranes plasmiques des cellules et constituent donc des composés de grand intérêt pour le traitement de la mucoviscidose.

Comme illustré par les exemples, ils sont particulièrement efficaces pour provoquer la re-localisation membranaire de la protéine delF508-CFTR dans la mucoviscidose où cette protéine est retenue dans le réticulum endoplasmique, et de restaurer ainsi sa capacité de transport trans-membranaire.

Lors de l'élaboration des médicaments, les principes actifs, utilisés en quantités thérapeutiquement efficaces, sont mélangés avec les véhicules pharmaceutiquement acceptables pour le mode d'administration choisi. Ces véhicules peuvent être solides ou liquides.

Ainsi pour une administration par voie orale, les médicaments préparés sous forme de gélules, comprimés, dragées, capsules, pilules, gouttes, sirops et analogues. De tels médicaments peuvent renfermer de 1 à 100 mg de principe actif par unité.

Pour l'administration par voie injectable (intraveineuse, sous-cutanée, intramusculaire), les médicaments se présentent sous forme de solutions stériles ou stérilisables. Ils peuvent être également sous forme de suspensions ou d'émulsions.

Les médicaments de l'invention sont plus particulièrement administrés sous forme d'aérosols.

Les doses par unité de prise peuvent varier de 1 à 50 mg de principe actif. La posologie quotidienne est choisie de manière à obtenir une concentration finale d'au plus 100 µM en dérivé de purines dans le sang du patient traité.

D'autres caractéristiques et avantages de l'invention seront donnés dans les résultats rapportés ci-après afin d'illustrer l'invention.

Dans ces exemples, il est fait référence aux figures 1 à 6, qui représentent
- la figure 1 : l'effet de la roscovitine sur l'adressage de delF508 dans les cellules CF15 (1A) ; sur l'activité de CFTR dans les cellules calu-3 (1B) ; sur l'adressage de CFTR dans les cellules Calu-3 (1C) ;
- la figure 2 : les résultats d'immunoprécipitation et d'analyse Western-Blot ;
- les figures 3A à 3C : l' immunolocalisation de delF508-CFTR après 2 h de traitement ou sans traitement ;
- les figures 4A et 4B : l'activation de delF508 dans les cellules CF15 après traitement avec CFTR-T1 (CFTR-T1 désignant la roscovitine) et la réponse à la roscovitine en fonction du temps d'incubation ;
- les figures 5A et 5B : le pourcentage d'activation en fonction de la concentration en roscovitine et le profil pharmacologique des canaux CFTR dans les cellules CF15 après incubation avec de la roscovitine ;
- la figure 6 : la compétition entre CFTR-T1 et la machinerie de la chaperonne du RE sur l'adressage de delF508-CFTR.

### MATERIEL ET METHODES

### M1. Culture cellulaire

Cellules CHO-WT : Les cellules CHO (Chinese Hamster Ovary) sont des fibroblastes qui ont été transfectés avec le gène du CFTR sauvage (CFTR-WT). Ces cellules vont donc surexprimer la protéine CFTR.

Milieu de culture : Milieu MEM alpha (GIBCO) + 7 % de sérum de veau foetal + 0,5 % de pénicilline/streptomycine + 100 µM de méthotrexate (améthoptérine, Sigma).

Cellules CF15 : Les cellules CF15 sont des cellules épithéliales humaines d'origine nasale qui expriment le gène ΔF508-CFTR.

Milieu de culture : Milieu DMEM + HAM F12 + 10 % de FCS + 0,6 % de pénicilline/streptomycine + facteurs de croissance (insuline 5 µg/ml, transferrine 5 µg/ml, épinéphrine 5,5 µM, adénine 0,18 mM, EGF 10 ng/ml, T3 2 nM, hydrocortisone 1,1 µM) .

Cellules Calu-3 : Les cellules Calu-3 sont des cellules épithéliales humaines d'origine pulmonaire qui expriment le gène du CFTR sauvage.

Milieu de culture : Milieu DMEM/F12 avec glutamax + 7 % de sérum de veau foetal + 1 % de pénicilline/streptomycine.

### M2. Immunomarquage

L'immunomarquage permet de visualiser la localisation cellulaire de la protéine CFTR grâce à un anticorps (Ac) primaire anti-CFTR, puis un anticorps secondaire anti-anticorps primaire marqué au fluorophore Cy3.

Les cellules sont préalablement ensemencées sur des lamelles dans du milieu de culture approprié. 3 lavages au TBS (NaCl : 157 mM, Tris base : 20 µM, pH 7,4) de 5 min chacun sont effectués. Les cellules sont alors fixées par ajout de TBS-paraformaldéhyde (3 %) pendant 20 min. Après 3 lavages au TBS (5 min), les cellules sont incubées avec du TBS-triton 0,1 % (10 min) qui permet la formation de trous dans la membrane cellulaire, puis 3 lavages au TBS sont à nouveau effectués avant la mise en présence des cellules avec le TBS-BSA 0,5 %-saponine 0,05 % pendant 1 h. Les cellules sont ensuite incubées avec l'anticorps primaire anti-C terminal CFTR (2 µg/ml) pendant 1 h. 3 lavages au TBS-BSA-saponine de 5 min chacun sont réalisés avant l'incubation avec l'anticorps secondaire GAM-cy3 (1/400) pendant 1 h. Suite à 2 lavages au TBS de 5 min, les noyaux sont marqués par incubation au Topro3 (1/1000) pendant 5 min. Enfin, les lamelles peuvent être montées sur lame après 3 derniers lavages au TBS de 5 min. Les lames sont observées au microscope confocal (Bio-Rad) grâce à une excitation au laser aux longueurs d'onde appropriées. Afin de différencier le marquage entre Cy3 et Topro3 la couleur de fluorescence de Topro3 a été changée en bleu (couleur des noyaux).

### M3. Efflux de radiotraceurs

Les mesures de transport d'ions chlorure dans les cellules ont été réalisées à l'aide de la technique des efflux d'iodure radioactif (Becq et al., 1999 ; Dormer et al., 2001). Le traceur (¹²⁵I) est incorporé dans le milieu intracellulaire. Puis, la quantité de radiotraceur qui sort de la cellule est comptée après l'ajout de différents agents pharmacologiques. L'iodure est utilisé comme un traceur du transport d'irons chlorure. ¹²⁵I a de plus l'avantage d'avoir une courte durée de vie comparée à celle d'autres marqueurs comme ³⁵Cl (1/2 vie respective : 30 jours et 300 000 ans).

Les cellules sont mises en culture sur des plaques 24 puits dans un milieu adéquat. 2 rinçages avec du milieu d'efflux (NaCl : 136,6 mM, KCl : 5,4 mM, KH₂PO₄: 0,3 mM, NaH₂PO₄: 0,3 mM, NaHCO₃: 4,2 mM, CaCl₂ : 1,3 mM, MgCl₂: 0,5 mM, MgSO₄ : 0,4 mM, HEPES : 10 mM, D-glucose : 5,6 mM) sont effectués afin d'éliminer les cellules mortes qui relarguent la radioactivité de façon anarchique. Puis, les cellules sont mises à incuber avec 500 µl de charge (1 µCi/ml de ¹²⁵INa) pendant 30 min pour les CHO-WT ou 1 h pour les CF15 et Calu-3. L'iodure s'équilibre de part et d'autre de la membrane cellulaire. Le robot (MultiPROBE, Packard) réalise les étapes suivantes : le milieu de charge est rincé avec du milieu d'efflux pour éliminer la radioactivité extracellulaire. Le surnageant est collecté toutes les minutes dans des tubes à hémolyse et le milieu est remplacé par un volume équivalent (500 µl). Les prélèvements des 3 premières minutes ne subissent pas l'addition de drogue, ils permettent d'obtenir une ligne de base stable, caractérisant la sortie passive des ions I. Les 7 prélèvements suivants sont obtenus en présence de la molécule à tester. A la fin de l'expérience, les cellules sont lysées par ajout de 500 µl de NaOH (0,1 N)/0,1 % SDS (30 min), ainsi, la radioactivité restée à l'intérieur de la cellule peut être déterminée. La radioactivité présente dans les tubes à hémolyse est comptée en coups par minute (cpm) grâce à un compteur gamma (Cobra II, Packard). Les résultats en cpm sont exprimés sous forme de vitesse de sortie d'iodure radioactif (R) d'après la formule suivante : R (min⁻¹)=[In(¹²⁵I t₁) -In (¹²⁵I t₂)]/(t₁-t₂) avec ¹²⁵I t₁ : cpm au temps t₁ ; ¹²⁵I t₂ : cpm au temps t₂. Ce flux d'iodure est représenté sous forme de courbe. Afin de quantifier la sortie d'iodure due à l'administration de la molécule testée, on calcule le flux relatif suivant qui permet de s'affranchir du flux de base : Vitesse relative (min⁻¹)=Rpic-Rbasal. Enfin, ces résultats sont normalisés pour pouvoir comparer l'effet des différentes drogues entre elles. Les résultats sont présentés sous la forme de moyenne +/- SEM. Le test statistique de Student est utilisé pour comparer l'effet des drogues aux contrôles (les valeurs correspondantes à P<0,01 sont considérées comme statistiquement significatives).

### M4. Test de cytotoxicité

Le test de toxicité au MTT est un test colorimétrique qui repose sur la capacité des déshydrogénases mitochondriales à métaboliser le MTT (sel de tétrazolium jaune) en formazan (pourpre). L'absorbance, proportionnelle à la concentration de colorant converti, peut être alors mesurée par spectrophotométrie. Les cellules sont mises à incuber sur des plaques 96 puits en présence de l'agent à tester pendant 2 h. 3 contrôles sont réalisés : 100 % cellules vivantes : cellules sans agent ; 0 % cellules vivantes : cellules laissées à l'air libre ; blanc : milieu sans cellule. Les cellules sont rincées avec du milieu RPMI sans rouge de phénol pour que la couleur du milieu n'interfère pas dans les mesures de l'absorbance. Puis, elles sont incubées pendant 4 h avec 100 µl de solution de RPMI supplémentée en MTT (0,5 mg/ml). Le milieu est alors éliminé, l'ajout de 100 µl de DMSO permet de solubiliser le colorant converti (formazan). L'absorbance est mesurée par spectrophotométrie à 570 nm (pourpre) ; 630 nm (bruit de fond). Afin de s'affranchir du bruit de fond, le calcul suivant est effectué : DO_{réelle}=DO₅₇₀ₙₘ-DO₆₃₀ₙₘ- Puis, les résultats sont normalisés par rapport aux contrôles (100 % et 0 % de cellules vivantes) et sont présentés sous forme de moyenne +/-SEM.

### RESULTATS

### R1. Effet de la roscovitine sur l'adressage de delF508 dans les cellules CF15

L'étude de l'adressage de la protéine delF508-CFTR est réalisée au laboratoire en combinant des approches de pharmacologie, d'imagerie cellulaire, des tests biochimiques et électrophysiologiques sur des cellules CF15 épithéliales humaines pulmonaires homozygotes pour la délétion delF508.

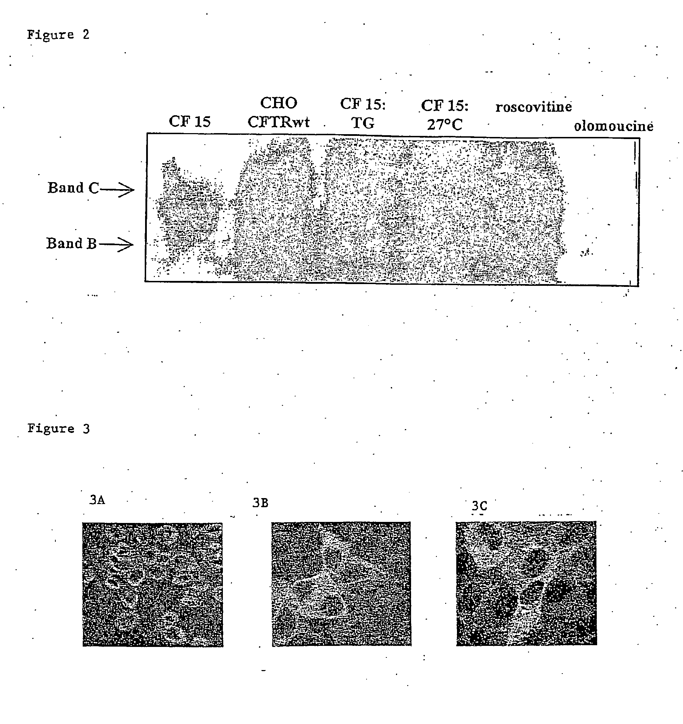

Les résultats d'immunoprécipitation et d'analyse Western-Blot de CFTR non muté de cellules CHO de type sauvage et de cellules delF508 traitées 2 h avec 100 µM de composé testé (figure 3C) ou de cellules non traitées (contrôle). Les cellules CF15 traitées 24 h à 27° C (figure 3B) ou 2h avec 10 µM de thapsigargin (TG) on été utilisées comme contrôle positif.

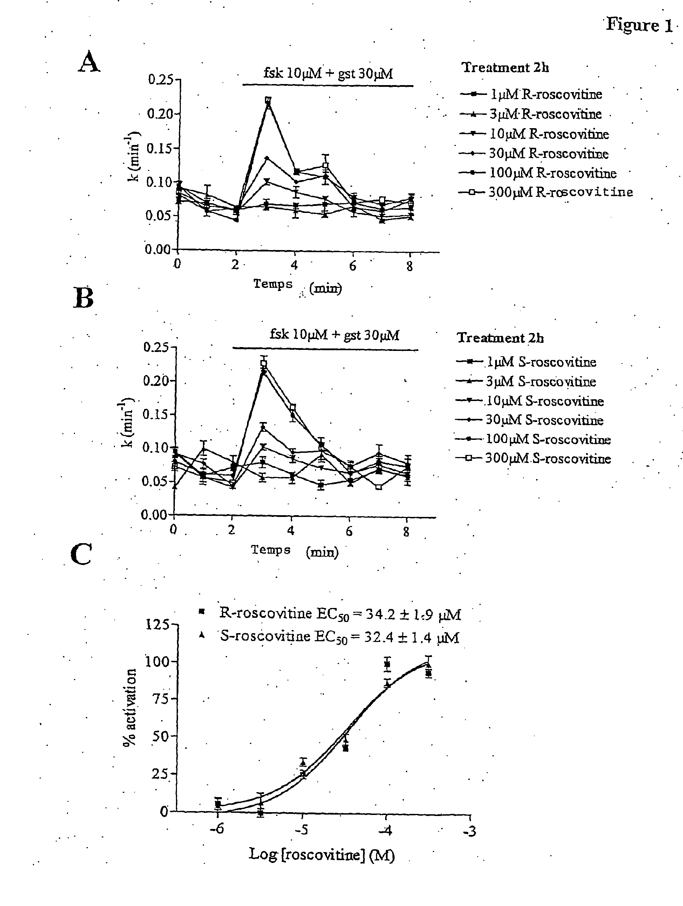

Pour chaque expérience, l'addition d'un cocktail (Forskoline 10 µM + Génistéine 30 µM) permet l'activation du CFTR quand celui-ci est localisé à la membrane. Ainsi, un efflux d'iodure pourra être observé si l'adressage de delF508 a été restauré. Les résultats, présentés sous forme d'histogramme ont été normalisés par rapport à un traitement de référence (traitement des cellules au MPB-91 250 µM pendant 2 h) pour lequel on considère qu'on a 100 % d'activité CFTR. Les résultats obtenus montrent que le traitement par les deux formes R et S de la roscovitine, des cellules CF15 pendant 2 h à 37 °C restaure un adressage de la protéine delF508 et permet à celui-ci de fonctionner en tant que transporteur ionique (figure 1).

En l'absence de traitement des cellules, la protéine delF508 n'est pas membranaire et il n'y a pas d'efflux d'iodure stimulé par le cocktail (Forskoline 10 µM, Génistéine 30 µM). L'EC₅₀ (concentration de la molécule qui donne 50 % de l'efficacité maximale) de la roscovitine a été déterminé à 34 ± 1,9 µM (R-Roscovitine, figure 1A, C) et 32 ± 1,4 (S-Roscovitine, figure 1B, C) (n=4, pour chaque condition). L'imagerie cellulaire permet de montrer que la protéine delF508 a été localisée dans les compartiments membranaires plasmatiques après traitement par la roscovitine.

Les figures 3A à 3C illustrent également l'immunolocalisation de delF508-CFTR après 2 h de traitement par la roscovitine ou en l'absence de traitement. Il s'agit d'une visualisation confocale de CFTR-delF508 dans des cellules CF15 avec un anticorps monoclonal anti-CFTR de souris. Les cellules CF15 traitées 24 h à 27°C ont été utilisées comme témoin positif (figure 3A).

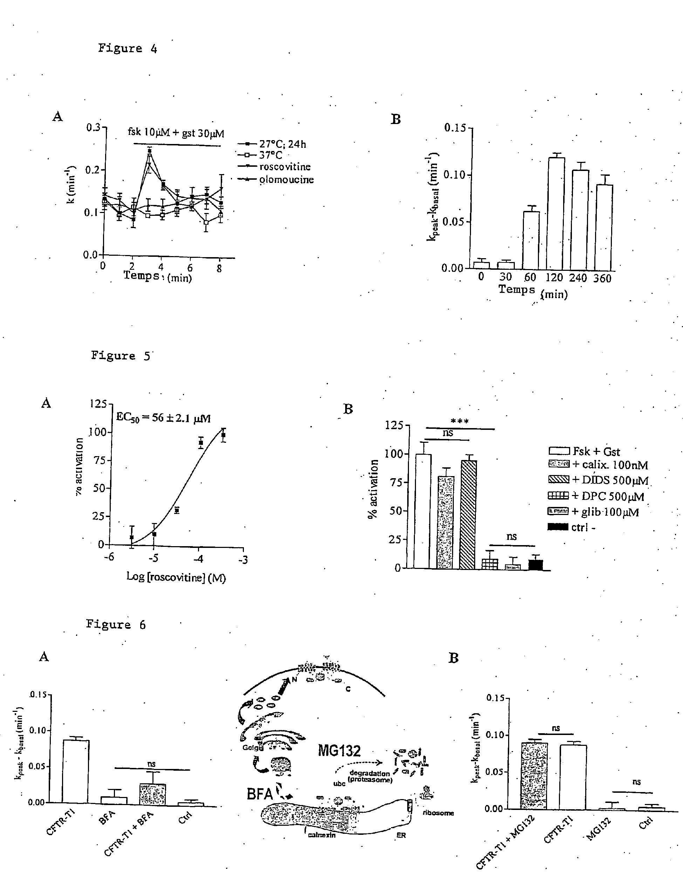

La figure 4A illustre l'activation de delF508-CFTR dans des cellules CF15 après traitement par la roscovitine.

Les efflux d'iodure ont été observés après 2 h d'incubation avec 100 µM de composé testé ou en l'absence de traitement.

Les cellules CF15 traitées 24 h à 27°C ont été utilisées comme contrôle positif et les cellules CF15 non traitées comme contrôle négatif (37°C).

La figure 4B montre que la réponse à la roscovitine dépend du temps d'incubation. 100 µM de roscovitine ont été utilisés pour traiter les cellules CF15. Les cellules ont été stimulées avec 10 µM fsk + 30 µM gst.

La figure 5A donne les résultats dose-réponse après 2 h de traitement avec la roscovitine. Dans la figure 5B, on rapporte les profils pharmacologique des canaux CFTR dans les cellules CF15 après 2 h d'incubation avec 100 µM de roscovitine.

Ces résultats montrent qu'après traitement par la roscovitine, on observe :
- une maturation de delF508-CFTR (voir bande C qui apparaît dans le Western-Blot) après traitement par CFTR-T1,
- une correction de la localisation de delF508-CFTR au niveau de la membrane plasmique, et
- une correction maximale de la fonction de canal chlorure de delF508-CFTR après 2 h de traitement.

La compétition entre CFTR-T1 et la machinerie de la chaperonne du RE est illustrée par les figures 6A et 6B. La figure 6A porte sur l'inhibition de l'action de CFTR-T1 par la Brefeldine A (BFA), inhibiteur du trafic vésiculaire ERGIC, ce qui montre que la roscovitine induit un réadressage de la protéine delF508-CFTR. La figure 6B montre qu'aucune modulation n'est observée en présence de MG132, un inhibiteur de protéasome, ce qui montre une compétition entre la roscovitine et MG132.

Le tableau ci-dessous donne un résumé d'expériences de compétition réalisées par la technique d'efflux d'iodure entre la roscovitine et la machinerie de la chaperone du RE.

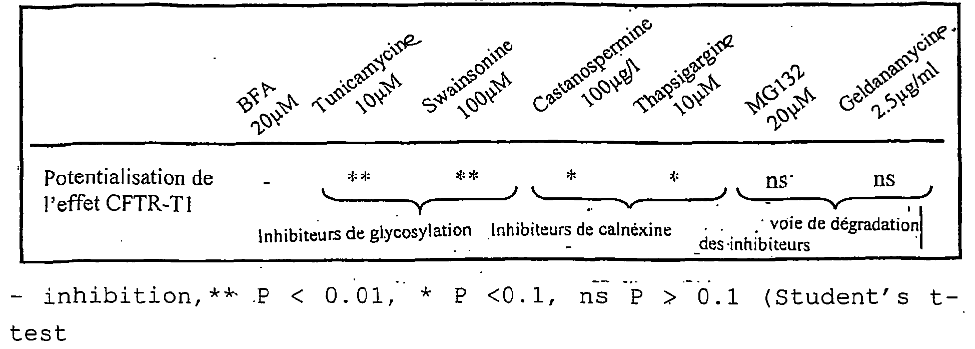

Ces résultats montrent que la roscovitine induit un réadressage de delF508-CFTR, inhibe la voie de dégradation de CFTR-T1 et peut moduler l'interaction entre CFTR-T1 et la calnexine (mécanisme calcium-dépendant).

### R2. Effet de la roscovitine sur l'activité de CFTR dans les cellules Calu-3

Afin de montrer que l'effet de la roscovitine est spécifique de l'adressage du delF508 et n'altère pas les autres canaux chlorure, la roscovitine a été testée en tant qu'activateur potentiel sur des cellules Calu-3. Ces résultats ont été obtenus en efflux iodure sur cellules Calu-3. **Les** contrôles **utilisés** sont la forskoline (5 µM, n=8) et le MPB-91 (250 µM, n=8) la roscovitine (n=8) n'est pas un activateur du CFTR sauvage ni d'autres transports anioniques de ces cellules (pas de différence significative).

### R3 . Effet de roscovitine sur l'adressage de CFTR dans les cellules Calu-3

Afin de montrer que l'effet de la roscovitine est spécifique de l'adressage du delF508, la roscovitine a été testée en tant que modulateur de l'adressage du CFTR sauvage sur des cellules Calu-3. Ces résultats ont été obtenus en efflux iodure sur cellules Calu-3 traitées 2 h par la roscovitine (100 µM). L'activité CFTR dans cette condition expérimentale n'est pas significativement différente par rapport aux contrôles. Ces résultats démontrent que la roscovitine n'affecte pas la voie d'adressage du CFTR sauvage ou d'autres canaux chlorure, ni n'altère l'activité de CFTR dans les cellules épithéliales humaines pulmonaires non-CF.

### R4. Cytotoxicité de la roscovitine

Dans le but de tester la cytotoxicité de la roscovitine, des cellules CHO-WT ont été incubées 2 h avec différentes concentrations en inhibiteurs avant de subir le test de viabilité cellulaire au MTT. Les résultats montrent que les cellules sont viables pour toutes les concentrations de la roscovitine. Cette molécule ne présente donc pas de cytotoxicité cellulaire.

Les tests d'efflux ont révélé que la roscovitine permet une relocalisation membranaire de la protéine delF508-CFTR et représente donc, un moyen pharmacologique de ré-adressage du delF508 dans une cellule épithéliale humaine pulmonaire.

Un traitement de 2 h par 100 µM de roscovitine entraîne l'apparition de la bande mature du CFTR (comme le montrent les techniques d'immunoprécipitation et de western-blot) indiquant que la roscovitine a permis la libération du RE, puis la maturation dans le Golgi de la protéine delF508-CFTR. Des expériences d'immunofluorescence ont confirmé qu'un traitement de 2h à 100 µM de roscovitine permettait la relocalisation membranaire de la protéine delF508-CFTR. Enfin, des expériences d'efflux d'iodure et de patch-clamp sur cellule entière ont mis en évidence la fonctionnalité de la protéine réadressée. Le traitement par la roscovitine présente une EC50 de 56 µM pour 2 h de traitement et permet un réadressage maximal après 2 h.

D'autre part, des expériences de compétition visant à déterminer le mécanisme d'action de la roscovitine ont permis de démontrer que la roscovitine entrait en compétition avec les inhibiteurs de la liaison calnexine/ delF508-CFTR ainsi qu'avec les inhibiteurs de la voie de dégradation

Ces différents résultats permettent de mettre en évidence que la roscovitine corrige le défaut d'adressage de la protéine delF508-CFTR. Un traitement par la roscovitine de cellules CF doit alors permettre d'interférer avec la capacité que possède la machinerie de contrôle d'interagir et de retenir la protéine delF508-CFTR dans le réticulum endoplasmique, ceci par l'intermédiaire d'une inhibition de la calnéxine et des molécules chaperonnes qui interviennent dans la voie de dégradation.

### Exemple de formulation

On prépare une solution pour inhalation avec un nébuliseur ampoule à partir de chlorure de sodium, de chlorure de calcium déshydraté et d'eau pour préparations injectables.

La roscovitine est ajoutée comme ingrédient actif.

La solution est formulée dans des ampoules de 2,5ml.

Des ampoules renfermant 5, 10 mg ou 20 mg roscovitine sont ainsi préparées.

### BIBLIOGRAPHIE

BECQ et al. (1999) Journal of Biological Chemistry 274, 27415-27425.
DORMER et al. (2001) Journal of Cell Science 114, 4073-4081.

## Revendications

1. Dérivés de purines pour leur utilisation dans le traitement de la mucoviscidose,
**caractérisés en ce que** lesdits dérivés sont choisis parmi la 2- (R, S) (1-éthyl-2-hydroxyéthylamino) -6-benzylamino-9- isopropylpurine, la 2- (R) - (1-éthyl-2-hydroxyéthylamino) - 6-benzylamino-9-isopropylpurine, et la 2-(S) (1- éthyl-2-hydroxyéthylamino) -6-benzylamino-9-isopropylpurine.

2. Dérivés pour leur utilisation selon la revendication 1, **caractérisés en ce que** les dérivés sont utilisés pour fabriquer des médicaments préparés pour une administration sous formes de gélules, comprimés, dragées, capsules, gouttes ou sirops.

3. Dérivés pour leur utilisation selon la revendication 1, **caractérisée en ce que** les dérivés sont utilisés pour fabriquer des médicaments préparés pour une administration par voie injectable, sous forme de solution.

4. Dérivés pour leur utilisation selon la revendication 1, **caractérisée en ce que** les dérivés sont utilisés pour fabriquer des médicaments préparés pour une administration sous forme d'aérosol.

## Patentansprüche

1. Purin-Derivate zur Verwendung bei der Behandlung von Mukoviszidose,
**gekennzeichnet dadurch, dass** die Derivate ausgewählt sind aus 2-(R,S)-(1-Ethyl-2-Hydroxyethylamino)-6-Benzylamino-9-Isopropylpurin, 2-(R)-(1-Ethyl-2-Hydroxyethylamino)-6-Benzylamino-9-Isopropylpurin, und 2-(S)-(1-Ethyl-2-Hydroxyethylamino)-6-Benzylamino-9-Isopropylpurin.

2. Derivate zur Verwendung gemäß Anspruch 1, **gekennzeichnet dadurch, dass** die Derivate zur Herstellung von Medikamenten verwendet werden, die zur Verabreichung in Form von Gelatinekapseln, Tabletten, Dragees, Kapseln, Tropfen oder Sirupen hergerichtet sind.

3. Derivate zur Verwendung gemäß Anspruch 1, **gekennzeichnet dadurch, dass** die Derivate zur Herstellung von Medikamenten verwendet werden, die zur Verabreichung auf dem Weg der Injektion in gelöster Form hergerichtet sind.

4. Derivate zur Verwendung gemäß Anspruch 1, **gekennzeichnet dadurch, dass** die Derivate zur Herstellung von Medikamenten verwendet werden, die zur Verabreichung in Form von Aerosol hergerichtet sind.

## Claims

1. Derivatives of purines for their use in the treatment of cystic fibrosis,
**characterised in that** these derivatives are selected from 2- (R, S) (1-ethyl-2-hydroxyethylamino) -6-benzylamino-9- isopropyl purine, 2- (R) - (1-ethyl-2-hydroxyethylamino) 6-benzylamino-9-isopropyl purine and 2-(S) (1-ethyl-2-hydroxyethylamino) -6-benzylamino-9-isopropyl purine.

2. Derivatives for their use according to claim 1, **characterised in that** the derivatives are used to produce medicines prepared for administration in the form of capsules, tablets, pills, drops or syrups.

3. Derivatives for their use according to claim 1, **characterised in that** the derivatives are used to produce medicines prepared for administration by injection in the form of a solution.

4. Derivatives for their use according to claim 1, **characterised in that** the derivatives are used to produce medicines prepared for administration in the form of a spray.
